# EUROPEAN PATENT APPLICATION

(11) **EP 1 829 549 A1**
(43) Date of publication of application: **05.09.2007**
(21) Application number: 05816911.1
(22) Date of filing: 16.12.2005
(51) Int. Cl.: A61K 38/00, A61K 9/14, A61K 9/20, A61K 9/70, A61K 47/38, A61P 25/04, A61P 43/00, C07D 417/12

(54) **AMORPHOUS COMPOSITION**

(30) Priority: 17.12.2004 JP 2004366081
(71) Applicant: ONO PHARMACEUTICAL CO., LTD., Osaka-shi, Osaka 541-8526 (JP)
(72) Inventor: MASUDA, Hideo, ONO PHARMACEUTICAL CO., LTD., Mishima-gun, Osaka 618-8585 (JP); SUGIHARA, Hikaru, ONO PHARMACEUTICAL CO., LTD., Mishima-gun, Osaka 618-8585 (JP); NISHIURA, Akio, ONO PHARMACEUTICAL CO., LTD., Mishima-gun, Osaka 618-8585 (JP)
(74) Representative: Henkel, Feiler & Hänzel
(86) International application number: PCT/JP2005/023140
(87) International publication number: WO 2006/064906

(57) **Abstract**

To provide an amorphous composition for nasal administration or for administration by adhering to oral mucosa in which absorption property and chemical and physical stabilities of (2R)-N-(1-benzylpiperidin-4-yl)-3-cyclohexylmethylthio-2-[(4R)-3-tert-butoxycarbonylthiazolidin-4-ylcarbonylamino]propanamide which is useful as an N type calcium channel inhibitor are improved.

A preparation comprising the amorphous composition of the present invention has been found to be excellent in physical stability and chemical stability and to be useful as a nasal preparation or a preparation for adhering to the oral mucosa. As a result, the resulting preparation has a high BA value and is useful for prevention and/or the treatment of a disease mediated by the N type calcium channel including pain (such as neuropathic pain, cancerous pain, intractable pain, postoperative pain, acute pain, chronic pain, neuralgia and infectious pain).

## Description

### Technical Field

The present invention relates to an amorphous composition.

The present invention relates to a pharmaceutical composition comprising amorphous (2R)-N-(1-benzylpiperidin-4-yl)-3-cyclohexylmethylthio-2-[(4R)-3-tert-butoxycarbonylthiazolidin-4-ylcarbonylamino]propaneamide or a salt thereof and a non-crystallizing polymer.

### Background Art

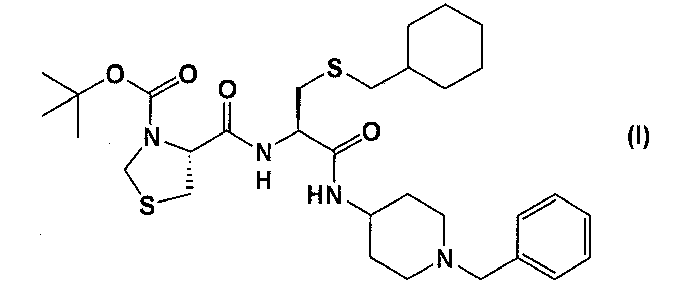

(2R)-N-(1-benzylpiperidin-4-yl)-3-cyclohexylmethylthio-2-[(4R)-3-tert-butoxycarbonylthiazolidin-4-yl-carbonylamino]propaneamide (CAS Registry No. 253306-39-7; hereinafter, it will be referred to as the compound (I)) represented by the above formula or a salt thereof has been known to have an N-type calcium channel inhibiting activity;, to be effective as an agent for preventing and/or treating cerebral infarction, transient cerebral ischemia attack, encephalomyelopathy after cardiac operation, vascular disease of spinal cord, stress hypertension, neurosis, epilepsy, asthma, pollakiuria and the like; and to be useful as an analgesic agent (refer to Patent Document 1).

The compound (I) or a salt thereof is also effective as an agent for preventing and/or treating diseases caused by retinal ischemia (such as glaucoma, diabetic retinopathy, macular degeneration and retinal vascular occlusion) (refer to Patent Document 2).

Since solubility of the compound (I) in water is as low as about 1.25 µg/mL, the compound (I) is not always suitable for an injection preparation. There is also a problem that, when the compound (I) is orally administered, its bioavailability (BA) is as very low as about 0.2%. Since the compound (I) is metabolized by CYP3A4 which is a metabolic enzyme, it is affected by a first-pass effect in the small intestine or in the liver and that is believed to be the cause for a low BA. Additionally, since the amount of a metabolic enzyme is greatly different among individuals, its oral administration results in a big difference concentration transition into serum among individuals. Thus, its use as an oral preparation is difficult.

Moreover, crystalline compounds are resistant to changes with lapse of time and are durable even for storage for long time, whereas their solubility in a solvent such as water in a crystalline state is not usually high that in an amorphous state.

On the other hand, when the compound is amorphous, its energy state is high and, therefore, it tends to unstable, whereas it is apt to become supersaturated when dissolved in a solvent such as water whereby the apparent solubility is generally high.

As described above, an amorphous form is usually with a high solubility unlike a crystal form, and is apt to be taken into living body. Therefore, a stable preparation which is able to maintain such an amorphous form for a long period is desirable in order to enhance the absorption of an agent.

In view of the above, there has been a great demand for the development of a stable amorphous pharmaceutical composition which is able to improve the problems described above.

On the other hand, there has been known a method where BA of a slightly soluble agent is improved as an orally administering agent using HPMCAS which is known as a substrate for pharmaceutical preparations (refer to Patent Document 3). Additionally, HPMCAS is usually used as an enteric polymer and there has been no report in which it is applied to a nasal preparation. As an administering method which is not affected by the first-pass effect, an approach where the compound (I) is used as a nasal preparation has been carried out (refer to Patent Document 4).
Patent Document 1: WO 2000/000470
Patent Document 2: WO 2002/051431
Patent Document 3: Japanese Patent No. 2,984,661
Patent Document 4: WO 2004/113332

### Disclosure of the Invention

### Problems to be solved by the Invention

An object of the present invention is to provide a novel pharmaceutical composition wherein the above-mentioned problems concerning the compound (I) or a salt thereof are solved.

### Means for Solving the Problems

In view of the above-mentioned problems, the inventors of the present invention carried out intensive studies. As a result, they have found that the resulting amorphous composition has good solubility and chemical and physical stabilities when a mixture of the compound (I) or a salt thereof with a non-crystallizing polymer is made into the amorphous composition. As a result of further investigations, they have also found that a preparation where BA is enhanced in the case of a nasal administration of the composition is achieved when the particle size is appropriately designed so that contact of the agent to the olfactory site of the nasal cavity is made minimum. Thus, the present invention has been accomplished.

Further as a result of the investigation for a preparation which is able to avoid the first-pass effect, it has been found that tablets where the amorphous composition comprising the compound (I) or a salt thereof and a non-crystallizing polymer is made into tablets or a film preparation where it is applied on a film is a preparation in which BA is unexpectedly enhanced by administration via adhering to oral mucosa. Thus, the present invention has been accomplished.

Accordingly, an object of the present invention is to provide the compound (I) or a salt thereof as an amorphous composition where chemical and physical stabilities are enhanced and, as a result, BA of the compound (I) is able to be enhanced.

Thus, the present invention relates to:
(1) A pharmaceutical composition comprising amorphous (2R)-N-(1-benzylpiperidin-4-yl)-3-cyclohexylmethylthio-2-[(4R)-3-tert-butoxycarbonylthiazolidin-4-ylcarbonylamino]propanamide or a salt thereof and a non-crystallizing polymer;
(2) The composition according to the above (1), wherein the non-crystallizing polymer is hydroxypropyl methylcellulose acetate succinate or hydroxypropyl cellulose;
(3) The composition according to the above (1), wherein the non-crystallizing polymer is hydroxypropyl methylcellulose acetate succinate;
(4) The composition according to the above (3), wherein it is a transnasal preparation;
(5) The composition according to the above (4), wherein it is powdery;
(6) The composition according to the above (5), wherein an average particle size of the powder is 75 µm to 180 µm;
(7) The composition according to the above (5), wherein an average particle size of the powder is 100 µm to 150 µm;
(8) The composition according to the above (3), wherein it is an oral transmucosal preparation;
(9) The composition according to the above (8), wherein it is an oral mucosal adhesive tablet or an oral mucosal adhesive film preparation;
(10) The composition according to the above (3), wherein hydroxypropyl methylcellulose acetate succinate is 100 to 350 parts by weight to 100 parts by weight of (2R)-N-(1-benzylpiperidin-4-yl)-3-cyclohexylmethylthio-2-[(4R)-3-tert-butoxycarbonylthiazolidin-4-ylcarbonylamino]propaneamide;
(11) The composition according to the above (3), wherein the rate of the amorphous substance after storing at 60°C for one month is 30% to 100%;
(12) The composition according to the above (3), wherein the residual rate of (2R)-N-(1-benzylpiperidin-4-yl)-3-cyclohexylmethylthio-2-[(4R)-3-tert-butoxycarbonylthiazolidin-4-ylcarbonylamino]propanamide after storing at 60°C for one month is 95% to 100%;
(13) The composition according to the above (3), wherein the bioavailability of (2R)-N-(1-benzylpiperidin-4-yl)-3-cyclohexylmethylthio-2-[(4R)-3-tert-butoxycarbonylthiazolidin-4-ylcarbonylamino]propanamide is 5% to 50%;
(14) The composition according to the above (3), wherein the rate of the amorphous substance after storing at 60°C for one month is 30% to 100%, the residual rate of (2R)-N-(1-benzylpiperidin-4-yl)-3-cyclohexylmethylthio-2-[(4R)-3-tert-butoxycarbonylthiazolidin-4-ylcarbonylamino]propanamide after storing at the same temperature for one month is 95% to 100% and its bioavailability is 5% to 50%;
(15) The composition according to the above (3), wherein it is a granular product prepared by spray-drying or a granular product prepared by stirring;
(16) The composition according to the above (3), wherein it is an agent for treating and/or preventing pain;
(17) The composition according to the above (16), wherein the pain is neuropathic pain, cancerous pain, intractable pain, postoperative pain, acute pain, chronic pain, neuralgia or infectious pain;
(18) A method for the stabilizing amorphous (2R)-N-(1-benzylpiperidin-4-yl)-3-cyclohexylmethylthio-2-[(4R)-3-tert-butoxycarbonylthiazolidin-4-ylcarbonylamino]propanamide using hydroxypropyl methylcellulose acetate succinate; and
(19) A method for producing a pharmaceutical composition comprising stable amorphous (2R)-N-(1-benzylpiperidin-4-yl)-3-cyclohexylmethylthio-2-[(4R)-3-tert-butoxycarbonylthiazolidin-4-ylcarbonylamino]propanamide, which comprises using hydroxypropyl methylcellulose acetate succinate.

The compound (I) or a salt thereof may be produced by a known process such as the process mentioned in the specification of WO 2000/00470.

In pharmaceuticals, stability is very important since it has a direct effect on efficacy and stability and there are many cases where shelf life of pharmaceuticals is usually requested to be three years or longer.

Stability usually means the degree where state is physically and chemically maintained. Stability greatly varies by affection of external environment such as temperature and humidity, by temperature dependency of the chemical reaction of the preparation *per se,* namely the extent of activation energy of the reaction, or by the temperature and humidity dependency of the permeability of material and container and the like.

Particularly, physical stability in the present invention means the degree where the compound (I) which is an active ingredient of the pharmaceutical is not crystallized but maintains an amorphous state.

In the present invention, chemical stability means the degree where the compound which is an active ingredient of the pharmaceutical does not change its chemical structure such as oxidation, rearrangement and dehydration but is maintained, in other words, the remaining degree (residual rate) of said compound after being stored under a predetermined condition for a predetermined period.

For example, a compound in a stable state such as in a crystal state is resistant to changes with lapse of time and is durable for storage during a long period. On the other hand however, solubility of a compound in a crystal state in a solvent such as water is not usually so high as an amorphous state.

Conversely, when a compound is amorphous, since its energy state is high, there is a tendency that it is unstable, while when it is dissolved in a solvent such as water, it tends to become a supersaturated state and an apparent solubility is usually high.

As described above, an amorphous form usually tends to be soluble unlike a crystalline form and tends to be taken in a living body. Therefore, in order to highly maintain the absorption of agent, a pharmaceutical preparation where such an amorphous form is able to be maintained is desirable.

On the other hand, since nerves in an olfactory site are uncovered, a transient strong stimulation is occurred when the preparation contacts the nerve in administration of a nasal preparation. Therefore, sprinkling distance of the preparation is controlled by controlling the particle size and the contact at the olfactory site is prevented beforehand to enable suppressing stimulation to be minimum and greatly improving the compliance of a patient with administration of the preparation. Incidentally the particle size by which the sprinkling distance is controlled and the stimulation is made to be minimum is also the particle size which is suitable for dissolution and absorption. Thus, it is possible that the stimulation is made to be minimum and BA is improved at the same time by controlling particle size.

Since a nasal preparation is able to avoid a first-pass effect, it contributes in improvement in BA more than an oral administration. At the same time, it can suppress non-uniform absorption of metabolic enzymes among individuals caused by the difference in gene. Additionally, since the blood level quickly rises after the administration, a prompt efficiency is able to be expected for pain (such as neuropathic pain, cancerous pain, intractable pain, postoperative pain, acute pain, chronic pain, neuralgia and infectious pain) which is a target for the treatment by the compound (I) or a salt thereof. Moreover, it is waterless upon administration. Therefore, since its handling is easy, it is also suitable for potion.

In view of the above, producing the compound (I) or a salt thereof into a nasal preparation is not only to solve the problems due to physical properties, but also to contribute in an improvement in drug taking compliance by a patient. Therefore, a very useful pharmaceutical preparation can be provided.

A method for avoiding the first-pass effect by oral administration includes an oral transmucosal preparation (such as an oral transmucosal tablet or an oral mucosal adhesive film).

The oral transmucosal preparation is a dosage form in which a preparation is adhered in an oral mucosa such as gingiva, back of the cheek or soft palate for the purpose of topical or systemic activity. The high BA can be expected in the preparation by avoidance of the first-pass effect. Additionally, being waterless in administration can keep high patient compliance. Furthermore, there is an advantage that no special device is needed. Accordingly, the oral transmucosal preparation of the compound (I) or a salt thereof is also able to be a useful pharmaceutical preparation.

"Non-crystallizing polymer" in the present invention includes everything which is able to make the compound (I) or a salt thereof to be amorphous. As the non-crystallizing polymer, for example, HPMCAS and HPC (hydroxypropyl cellulose) is preferable and HPMCAS is particularly preferable.

"HPMCAS" used in the present invention means a cellulose derivative which is able to have (1) two types of ether substituents (methyl and/or 2-hydroxypropyl) and (2) two types of ester substituents (acetyl and/or succinyl), which is chemically expressed as O-(2-hydroxypropyl)-O-methyl-cellulose acetate succinate.

Commercially available HPMCAS may be used. The commercially available HPMCAS includes Shin-Etsu AQOAT-LF, Shin-Etsu AQOAT-MF, Shin-Etsu AQOAT-HF, Shin-Etsu AQOAT-LG, Shin-Etsu AQOAT-MG and Shin-Etsu AQOAT-HG (all are trade names; manufactured by Shin-Etsu Chemical Co., Ltd.). HPMCAS is able to be purchased from many makers, but may produce, for example, by the treatment of O-(hydroxypropyl)-O-methylcellulose with acetic anhydride and succinic anhydride (refer to Carbohydrate, 222, (1991), 255-259 and U. S. Patent No. 4, 385, 078).

"HPC" used in the present invention means hydroxypropyl cellulose and is a nonionic cellulose ether produced by a manner that cellulose (pulp) which is widely present in nature is used as a material and is obtained react with an etherizing agent such as propylene oxide after treatment with sodium hydroxide. HPC includes, for example, HPC-SSL, HPC-SL, HPC-L, HPC-M, and HPC-H, and *the* like. All of them are able to be commercially available.

In the present invention, for example, when a mixture of the compound (I) or a salt thereof and HPMCAS is dissolved in a solvent followed by being spray-dried and being granulated, the amorphous state (i.e., physical stability) of the compound (I) along with its chemical stability is able to be maintained for a long period. HPMCAS forms a solid solution in a mixture with the compound (I) or a salt thereof and thus can keep the amorphous state.

Although the compound (I) is hardly soluble in water, its solubility in water rises under an acidic condition. It would appear that local pH decreases and agent solubility rises by being composed (i.e., a solid solution) with HPMCAS which is an acidic polymer. Solubility of crystals of the compound (I) in water is about 1.25 µg/mL while that of an amorphous composition prepared by granulating the compound with HPMCAS (the powder prepared by spray-drying in the Preparation Example 3) is about 30.3 µg/mL. The apparent solubility rises about 20-fold or more.

With regard to the physical stability, a combination of HPMCAS with the compound (I) or a salt thereof is able to maintain the amorphous state for a long period (refer to Test Examples 1 and 3) .

With regard to the chemical stability, degradation hardly happens in view of agent content, number of impurities and the like after elapse of certain period, and stability is improved (refer to Test Examples 1 and 3).

Accordingly, as a result of use of HPMCAS, the compound (I) is able to be made stable in amorphous state and an amorphous preparation containing the compound (I) as an active ingredient using HPMCAS is stable.

In the present invention, a symbol " 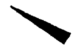 " means that it is bound to this side (β position) of the paper unless otherwise mentioned as it is apparent for persons skilled in the art, while a symbol " 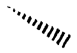 " means that it is bound to the other side (α position) of the paper unless otherwise mentioned. A symbol " 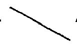 " means that it is a mixture in any ratio of the compounds binding to β position and to α position.

A salt of the compound (I) includes pharmaceutically acceptable salts, which are able to be produced by a known method.

The pharmaceutically acceptable salt in the present specification includes alkali metal salt, alkali earth metal salt, ammonium salt, amine salt, acid addition salt and the like.

With regard to the salt, those which have little toxicity and are soluble in water are preferable. Examples of the appropriate ones are salts of alkali metal (such as potassium and sodium), salts of alkali earth metal (such as calcium and magnesium), ammonium salt and salts with pharmaceutically acceptable organic amine (such as tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine and N-methyl-D-glucamine).

With regard to an acid addition salt, those which have little toxicity and are soluble in water are preferable. Examples of an appropriate acid addition salt are an inorganic acid salt such as hydrochloride, hydrobromide, hydroiodide, sulfate, phosphate and nitrate; and an organic acid salt such as acetate, lactate, tartrate, benzoate, citrate, methanesulfonate, ethanesulfonate, benzenesulfonate, toluenesulfonate, isethionate, glucuronate and gluconate.

The compound (I) or a salt thereof is also able to be converted to a solvate by a known method.

With regard to the solvate, those which have little toxicity and are soluble in water are preferable. An example of the suitable solvate includes solvates with water and an alcohol type (such as ethanol).

The amorphous composition of the present invention can be prepared using the compound (I) or a salt thereof and a non-crystallizing polymer such as HPMCAS an HPC. It is also possible that a pharmaceutically acceptable additive is appropriately mixed therewith. The pharmaceutically acceptable substrate and/or additive include various kinds of organic or inorganic substances which have been commonly used as materials for pharmaceutical preparations. They are, for example, excipient, lubricant, binder, disintegrating agent, thickener, suspending agent, emulsifying agent, isotonizing agent, buffer, soothing agent and stabilizer. If necessary, it is also possible to use other additives such as preservative (antiseptic agent), pH adjusting agent, refreshing agent, antioxidant and moisturizer.

Furthermore, followings are examples of an additive which may be added in the pharmaceutical preparation of the present invention, if necessary.

An example of an excipient includes lactose, white sugar, D-mannitol, starch, corn starch, crystalline cellulose, light anhydrous silicic acid and the like.

An example of a lubricant includes magnesium stearate, calcium stearate, talc, colloidal silica and the like.

An example of a binder includes crystalline cellulose, white sugar, D-mannitol, dextrin, hydroxypropyl cellulose, hydroxypropyl methylcellulose, polyvinylpyrrolidone, starch, sucrose, gelatin, methyl cellulose, carboxymethyl cellulose sodium and the like.

An example of a disintegrating agent includes starch, carboxymethyl cellulose, carboxymethyl cellulose calcium, croscarmellose sodium, carboxymethyl starch sodium, L-hydroxypropyl cellulose and the like. An example of a thickener includes polyalcohol such as glycerol and macrogol; celluloses such as methyl cellulose, carboxymethyl cellulose and hydroxypropyl methylcellulose; hydrophilic polymers such as polyvinyl alcohol, polyvinylpyrrolidone, carboxyvinyl polymer, carboxymethyl cellulose sodium, methylcellulose, hydroxymethyl cellulose, hydroxyethyl cellulose and hydroxypropyl cellulose; sodium alginate; chondroitin sulfate; cyclodextrin, d-α-tocopheryl polyethylene glycol 100 succinic acid; polyethylene glycol; and the like.

An example of a suspending agent includes a surfactant such as stearyl triethanolamine, sodium laurylsulfate, laurylaminopropionic acid, lecithin, benzalkonium chloride, benzethonium chloride, glycerol monostearate, polyoxyethylene hydrogenated castor oil and polysorbate; a polyalcohol such as glycerol and macrogol; saccharide such as sorbitol, mannitol and sucrose; celluloses such as methylcellulose, carboxymethyl cellulose and hydroxypropyl methylcellulose; a hydrophilic polymer such as polyvinyl alcohol, polyvinylpyrrolidone, carboxyvinyl polymer, carboxymethyl cellulose sodium, methylcellulose, hydroxymethyl cellulose, hydroxyethyl cellulose and hydroxypropyl cellulose; chondroitin sulfate; and the like.

An example of a soothing agent includes benzyl alcohol, chlorobutanol, propylene glycol, ethyl aminobenzoate, lidocaine, and the like.

An example of a stabilizer includes a sulfur compound such as sodium sulfite, sodium hydrogen sulfite, sodium hydrogen metasulfite, sodium thiosulfate, rongalite, thioglycerol, thioglycolic acid, thiolactic acid, cysteine, glutathione, thioacetic acid, methionine, thiosorbitol, thioglucose and thiourea; an inorganic acid and a salt thereof such as boric acid, borax, phosphoric acid, metaphosphoric acid, sodium carbonate and sodium hydrogen carbonate; an organic acid and a salt thereof (such as sodium edetate) such as formic acid, oxalic acid, tartaric acid, citric acid and edetic acid; an acid amide such as acetamide, diethyl acetamide, nicotinamide, urea barbital; urea derivatives; a polyalcohol such as glycol, propylene glycol, glycerol, polyethylene glycol, glucose and ascorbic acid; saccharides; phenols such as phenol, thymol, quinone, coumarone and isocoumarone; dibutylhydroxytoluene; an amino acid such as glycine, glutamic acid, lysine, phenylalanine, casein and edestin; proteins and the like.

An example of an emulsifier includes glycerol ester (such as glycerol monooleate), saponin (such as saponin of *Sophora japonica,* a quillaia extract and soybean saponin), sucrose fatty acid ester, lecithin (such as vegetable lecithin, egg yolk lecithin and soybean lecithin), polyalcohol (such as oleylalcohol, stearyl alcohol, cetylalcohol), fatty acid ester (such as octyldodecyl myristate), medium-chain fatty acid triglyceride (MCT), various kinds of surfactants (such as an emulsifier of an alkyl benzenesulfonate type, benzalkonium chloride, sorbitan sesquioleate and dodecyl benzene sulfonate), triethanolamine and the like.

An example of a preservative (antiseptic agent) includes a p-oxybenzoate ester such as propyl p-oxybenzoate and butyl p-oxybenzoate; parabens such as methyl paraben, ethyl paraben, propyl paraben and butyl paraben; invert soap such as benzalkonium chloride, benzethonium chloride, chlorhexidine gluconate and cetylpyridinium chloride; alcohol derivatives such as chlorobutanol, benzyl alcohol and phenethyl alcohol; an organic acid and derivatives thereof such as sodium dehydroacetate, sorbic acid and sodium sorbate; and phenols such as p-chloromethoxyphenol and p-chloro-m-cresol and the like.

An example of a pH adjusting agent includes sodium hydroxide, potassium hydroxide, trisodium phosphate, disodium hydrogen phosphate, hydrochloric acid, nitric acid, citric acid, boric acid, acetic acid, a phosphate buffer and the like.

An example of a refreshing agent includes 1-menthol, dl-menthol, camphor, peppermint water and the like.

An example of an antioxidant includes sulfites, ascorbic acid, citric acid, sodium edentate and the like.

An example of a moisturizer includes propylene glycol, polysorbate, macrogol, glycerol and the like.

An example of an adhesive includes hydroxypropyl cellulose, hydroxypropyl methylcellulose, carboxyvinyl polymer, polyethylene oxide and the like.

The amorphous composition of the present invention is preferably to be a solid agent or to be powdery. With regard to the additive used in the amorphous composition of the present invention, a binder and any of crystalline cellulose, white sugar, D-mannitol, dextrin, hydroxypropyl cellulose, hydroxypropyl methylcellulose, polyvinylpyrrolidone, starch, sucrose, gelatin, methylcellulose and carboxymethyl cellulose may be used preferably. Hydroxypropyl methylcellulose is more preferable. In the present invention, an amorphous composition can be prepared by, for example, a spray drying method wherein a mixture of the compound (I) or a salt thereof and a non-crystallizing polymer is made into amorphous.

A spray drying method is also called a spray-dry method and, although it is clear for persons skilled in the art, it stands for a process where a liquid mixture is made into small droplets (making into fine particles) and a solvent is quickly removed from the mixture in a container (spray drying device).

In the present invention, a method for making the compound (I) amorphous is not limited to a spray drying method but it also includes a method where a solvent is distilled using an evaporator, a heating/melting method, an extruding method, a supercritical method, a mixing/crushing method, an adsorbing method and the like.

An amorphous composition prepared by such a method may be uniformly dispersed in or adhered and bound to, for example, a solid physiologically acceptable additive or may be subjected to granulation to give a pharmaceutical preparation.

A method for granulation is that, for example, an amorphous composition comprising the compound (I) or a salt thereof and the non-crystallizing polymer together, which is further uniformly mixed with appropriate additives such as excipient, binder or disintegrating agent if necessary is made into granules by an appropriate method such as an extrusion granulation (granulation using an extruder), an extrusion granulation with heating, a granulation method with stirring (such as a mixing/stirring granulation method or a high-speed mixing/stirring granulation method), a fluidized-bed granulation method, a rolling-stirring fluidized-bed granulation method, a rolling granulation method, a dry (compression) granulation method, a crushing granulation method, a spray-drying granulation method and the like, and the resulting one is made into powder or fine particles by drying if necessary, followed by sieving so that the particle size is made as uniform as possible.

The amorphous composition of the present invention may be further coated using a coating agent. Examples of the coating agent are a dispersion of a copolymer of ethyl acrylate with methyl methacrylate, copolymers of aminoalkyl methacrylates, ethyl cellulose, carboxymethyl ethyl cellulose, dry methacrylate copolymer LD, cellulose acetate phthalate, a copolymer of dimethylaminoethyl methacrylate with methyl methacrylate, stearic acid, hydroxypropyl methylcellulose phthalate, starch partially made into an α-form, pullulan, polyoxyethylene (105) polyoxypropylene (5) glycol, polyvinyl alcohol, copolymers of methacrylates, magnesium metasilicate aluminate and the like.

The amorphous composition of the present invention can be produced preferably by spray-drying of a solution of a mixture of the compound (I) or a salt thereof and HPMCAS in water and/or an organic solvent (ethanol, acetone or isopropanol and the like; preferably, a mixture of ethanol and acetone) and by granulating the resulting powdery amorphous composition.

In making the amorphous composition of the present invention into a pharmaceutical preparation, it is preferable that the compound (I) is included in an amount of, for example, about 0.01 to about 60% or, preferably, about 3 to about 50% by weight in the pharmaceutical preparation.

In the amorphous composition of the present invention, the amount of the HPMCAS to 100 parts by weight of the compound (I) is preferably about 100 to about 350 parts by weight and, more preferably, about 150 to about 300 parts by weight.

By carrying out an investigation for achieving a good absorption in nasal cavity, it was found that its absorption pattern in nasal cavity was able to be made constant when an average particle size of the amorphous composition of the present invention was appropriately adjusted, which results in high BA. Specifically, it is preferable about 50 to about 300 µm, more preferable about 50 to about 200 µm, still more preferable about 75 to about 180 µm and, particularly preferable, about 100 to about 150 µm. When a high BA is achieved by making into a pharmaceutical preparation, it is possible to reduce the dose of the agent and it is supposed that drug-taking compliance of the patient is enhanced as well.

An average particle size is a particle size which represents a particle group when the particle group is constituted from many non-uniform particles. In an average particle size, there are weighted averages such as number-average, length-average, area-average or volume-average and an average surface area diameter or an average weight diameter wherein a diameter of imaginary sphere having average surface area and average volume of particles is taken into consideration.

The amorphous composition of the present invention also includes those where homogeneity is able to be ensured within such an extent that the object and the effect of the present invention are achieved. For example, in a composition comprising the compound (I) or a salt thereof and a non-crystallizing polymer, it is preferable that physical stability (such as an amorphous state) is able to be maintained for a long period. For instance, the rate of the amorphous substance to the whole composition after storing at 60°C for one month is preferably about 30 to about 100%, more preferably about 50 to about 100% and, still more preferably, about 70 to about 100%. It is further preferred that a predetermined rate of the amorphous state is able to be maintained even after, for example, three months, six months, one year, two years or three years under a certain condition.

In the present invention, it is preferable that chemical stability of the compound (I) or a salt thereof is maintained for a long period and, for example, the residual rate of the compound (I) or a salt thereof after storing at 60°C for one month is preferably about 90 to about 100%, more preferably about 95 to about 100% and, still more preferably, about 97 to about 100%. It is further preferable that a predetermined residual rate is able to be maintained even after, for example, three months, six months, one year, two years or three years under a certain condition.

The bioavailability (BA) of the compound (I) or a salt thereof when the amorphous composition of the present invention is administered to mammals such as human is preferably about 2 to about 60%, more preferably about 5 to about 50% and, still more preferably, about 7 to about 40%. With regard to a method of administration, nasal administration and administration by adhering to oral mucosa is preferable.

In making the amorphous composition of the present invention into a pharmaceutical preparation, it is also possible to add a smell corrigent having a masking effect to eliminate the smell derived from the compound or having an aromatic effect where the smell is masked by a stronger smell than that derived from an agent to enhance the compliance of the patient.

An example of a smell corrigent having a masking effect or, in other words, a masking agent includes trehalose, malic acid, maltose, potassium gluconate, essential oil of anis, essential oil of vanilla and essential oil of cardamom.

An example of a smell corrigent having an aromatic effect or, in other words, an aromatizing agent includes galenical components (such as cinnamon powder, peppermint powder, camphor powder, fennel powder, ginger powder, rosemary powder and perilla leaf powder), natural aroma oil or extract (such as peppermint oil, spearmint oil, Japanese mint oil, bergamot oil, tangerine oil, ylang-ylang oil, rose oil, geranium oil, orange extract, turpentine oil, clove oil, lemon powder, vanilla essence, peppermint essence and eucalyptus oil), various aromatic components (such as 1-menthol, dl-menthol, camphor, vanillin, limonene, butanol, isobutyl alcohol, hexanol, hexanal, trans-2-hexenal, cinnamic alcohol, phenylpropyl alcohol, cis-3-hexenol, ethyl butyrate, butyl acetate, butyl butyrate, irone, benzyl alcohol, linalool, geraniol, tagetone, dihydrotagetone, 3-methyl-5-(2-methylpropyl)-2-furan carboaldehyde, benzyl acetate, p-methylanisole, methyl benzoate, benzyl benzoate, linalyl acetate, nerolidol, nerol, indole, β-ionone, γ-decalactone, linalool oxide, methyl cinnamate, methyl anthranilate, cinnamic aldehyde, benzaldehyde, eugenol, phenylethyl alcohol, benzyl salicylate, citronellol, 1-hexadecene, anisaldehyde, palmityl aldehyde, anisilic acid, enanthic acid, caryophyllene, terpineol, γ-terpinene, lilac alcohol, α-pinene, ocimene, methyl benzyl ether, hydroquinone dimethyl ether, anisaldehyde, phytol, methyl jasmonate, cis-jasmone, rose oxide and damascenone).

In the present invention, surface of a pharmaceutical preparation is able to be treated with a masking agent (smell corrigent) or it is also possible that an extract per se which is powdery or liquid and has an aromatic effect before preparing into a pharmaceutical preparation is kneaded followed by making into the preparation.

With regard to a blending quantity of the compound (I) or a salt thereof in the amorphous composition of the present invention, although it may be selected depending on an amount which is necessary for activity and for treatment, it is preferable taking the fact that the administered compound is not usually completely absorbed in composition of the unit dose, namely, the BA of the compound into consideration. Additionally, for example, when multiple administrations are conducted in various dosage forms from the same container, it is preferable that a dose per one administration is the common dose or more.

When the amorphous composition of the present invention is nasally administered to mammals such as human, it is possible to use a widely used spray-drier for nasal powder such as Puvlizer (Teijin), Insaflator (Fisons), Jetlyzer (Hitachi) and Vidose (Pfeiffer). In that case, the dose of the compound (I) or a salt contained in the composition to human is about 5 to about 15 mg/human. More preferably, it is about 10 to about 90 mg/human and, still more preferably, it is about 15 to about 50 mg/human.

The amorphous composition of the present invention may be also compressed into tablets to be an oral mucosal adhesive tablets. The oral mucosal adhesive tablet is a tablet which is adhered to gingiva, back of the cheek, soft palate and the like expecting its topical or systemic activity with a purpose of absorption from oral mucosa.

In such an oral mucosal adhesive tablet, an amorphous composition which is a mixture of the compound (I) or a salt thereof and HPMCAS may be further mixed with a excipient (such as lactose, mannitol, glucose, finely crystalline cellulose and starch), a binder (such as hydroxypropyl cellulose, polyvinylpyrrolidone and magnesium metasilicate aluminate), a disintegrating agent (such as calcium cellulose glycolate), a lubricant (such as magnesium stearate), a stabilizer, a dissolving aid (such as glutamic acid and aspartic acid)and the like. It is made into tablets by a conventional method and used as a pharmaceutical preparation. If necessary, it may be coated with a coating agent (such as white sugar, hydroxypropyl cellulose and hydroxypropyl methyl cellulose phthalate) or may be coated with two or more layers.

The amorphous composition of the present invention may also be applied to or embedded in a substrate to give an oral mucosal adhesive film preparation. The oral mucosal adhesive film preparation is a preparation where active ingredient is applied to or embedded in a substrate and is fixed to gingiva, back of the cheek, soft palate and the like. expecting topical or systemic action with a purpose of absorption from oral mucosa.

In such an oral mucosal adhesive film preparation, the amorphous composition which is a mixture of the compound (I) or a salt thereof and HPMCAS is able to be applied to or embedded in the substrate polymer. A preferable example of the substrate polymer inclides carboxyvinyl polymer, carboxymethyl cellulose sodium, carrageenan, sodium alginate, propylene glycol alginate, xanthan gum, polyacrylic acid, sodium polyacrylate, tare gum, guar gum, locust bean gum, hydroxyethyl cellulose, hydroxypropyl cellulose, hydroxypropyl methylcellulose, methylcellulose, gellan gum, gelatin, curdlan, gum arabic, agar, pectin, polyvinyl alcohol and pullulan. If necessary, it is also possible to use the aforementioned additive such as excipient, lubricant, binder, disintegrating agent, thickener, suspending agent, emulsifying agent, isotonizing agent, buffer, soothing agent, stabilizer, preservative (antiseptic), pH adjusting agent, refreshing agent, antioxidant, moisturizer and smell corrigent (masking agent).

The oral mucosal adhesive preparation is a preparation wherein the QOL is enhanced to be easily administered to small children and aged people who have a low swallowing ability.

When the amorphous composition of the present invention is made into an oral mucosal adhesive tablet or an oral mucosal adhesive film preparation for mammals such as human, a dose of the compound (I) or a salt thereof contained in the whole composition to human is about 1 to 150 mg/human. More preferably, it is about 5 to 90 mg/human and, still more preferably, it is about 10 to 50 mg/human. This preparation is able to be administered for one to five times a day.

As shown in the following Preparation Examples and Test Examples, the amorphous composition of the present invention has been found to show a very high BA and to have excellent properties.

### Toxicity

Toxicity of the amorphous composition of the present invention is very low and is sufficiently safe to be used as a pharmaceutical.

### Application to Pharmaceuticals

In the amorphous composition of the present invention, BA of the compound (I) or a salt thereof is improved to be able to reduce the dose. As a result, it is able to be safely administered as a pharmaceutical. Moreover, the compound (I) shows an excellent N type calcium channel inhibiting activity to mammals, particularly to human. Therefore, it is useful as an agent for preventing and/or treatingdiseases mediated by an N type calcium channel such as pain (such as neuropathic pain, cancerous pain, intractable pain, postoperative pain, acute pain, chronic pain, neuralgia and infectious pain), cerebral infarction, transient cerebral ischemia attack, encephalomyelopathy after operation of the heart, vascular disease of spinal cord, stress hypertension, neurosis, epilepsy, asthma, pollakiuria and ocular diseases (such as glaucoma, diabetic retinopathy, macular degeneration and retinal vascular occlusion).

An example of neuropathic pain includes postherpetic neuralgia (such as pain after herpes), diabetic neuropathy, AIDS pain, trigeminal neuralgia and neuropathic back pain.

The amorphous composition of the present invention may be used as a concomitant agent in combination with other agents for 1) supplement and/or enhancement of preventing and/or treating effect of the compound (I) or a salt thereof; 2) improvement of kinetics and absorption and for reduction of dose of the compound (I) or a salt thereof; and/or 3) reduction of side effects of the compound (I) or a salt thereof.

The concomitant agent of the amorphous composition of the present invention with other agent may be administered in a form where both components are included in one nasal preparation or may be administered in a form of different preparations. The administration in a form of different preparations includes the administration at the same time and the administration at different times. In the case of the administration at different times, the preparation of the present invention may be firstly administered and other agents may be later or other agent(s) may be firstly administered and the preparation of the present invention may be administered later. Additionally, administering methods of them may be same or different. For example, with regard to the administering method for other agent(s), oral administration, parenteral administration (such as instillation, inhalation, fomentation and attaching)and the like may be preferably used.

Such other agent(s) may be a low-molecular compound or may be a high-molecular one such as protein, polypeptide, polynucleotide (DNA, RNA and gene), antisense, decoy, antibody or vaccine. Dose of other agent(s) may be appropriately selected based on a clinically used dose. Compounding ratio of the compound of the present invention to the other agent(s) may be appropriately selected depending on age and body weight of an object to be administered, administering method, administering time, object diseases, symptoms, combination and the like. For example, 0.01 to 100 part(s) by weight of the other agent(s) may be used to 1 part by weight of the compound of the present invention. With regard to the other agent(s), one or more member(s) freely selected from the following same group or different groups may be appropriately combined and administered.

There is no particular limitation for the disease for which the aforementioned concomitant preparation achieves preventing and/or treating effect. It can be any disease will do so far as it supplements and/or potentiates the preventing and/or treating effect of the compound of the present invention.

For example, an example of other agent(s) which supplements and/or potentiates the effect for pain by the compound (I) or a salt thereof includes narcotic or non-narcotic analgesic agent, nonsteroidal anti-inflammatory agent, antipyretic analgesic agent, antiepileptic agent, anti-arrhythmic agent, antidepressant, antianxiety agent, antipsychotic agent, adrenocortical hormone, antihistamine, local anesthetic, NMDA antagonist, treating agent for migraine, treating agent for painful diabetic neuropathy, calcium channel inhibitor and the like.

An example of a narcotic or non-narcotic analgesic agent includes opium, diluted powder of opium and ipecac, opium chloride alkaloid, opium alkaloid with atropine, opium alkaloid with scopolamine, morphine hydrochloride, morphine with atropine, ethylmorphine hydrochloride, compound oxycodone, compound oxycodone with atropine, codeine phosphate, dihydrocodeine phosphate, oxymethebanol, cocaine hydrochloride, pethidine hydrochloride, fentanyl citrate, pentazocine, pentazocine hydrochloride, tramadol hydrochloride, butorphanol hydrochloride, buprenorphine hydrochloride, eptazocine hydrobromide and the like.

An example of a nonsteroidal anti-inflammatory agent includes salsalate, sodium salicylate, aspirin, aspirin compounded with a dialuminate, diflunisal, suprofen, ufenamate, dimethylisopropylazulene, bufexamac, felbinac, tolmetin sodium, clinoril, napmeton, ibuprofen piconol, ketophenylbutazone, oxyphenbutazone, Napageln ointment, sulpyrine, migrenin, salidon, Sedes G, Amipylo-N, solbone, agent for cold of a pyrine type, acetaminophen, phenacetin, dimethothiazine mesylate, simetride-compound agent, remedy for cold of a non-pyrine type, salicylamide, fluphenamic acid, aluminum fluphenamate, mephenamic acid, aluminum mephenamate, floctafenine, tolfenamic acid, diclofenac, diclofenac sodium, sulindac, fenbufen, amfenac sodium, indomethacin, indomethacin farnesyl, proglumetacin maleate, acemetacin, nabumetone, etodolac, mofezolac, ibuprofen, ketoprofen, flurbiprofen, flurbiprofen axetil, oxaprozin, fenoprofen calcium, tiaprofenic acid, naproxen, pranoprofen, loxoprofen sodium, aluminoprofen, zaltoprofen, bucolome, piroxicam, ampiroxicam, tenoxicam, epirizole, tiaramide hydrochloride, emorfazone and the like. An example of antipyretic analgesic agent includes sulpyrine, acetaminophen, dimethothiazine mesylate and the like.

An example of an anti-epileptic agent includes phenytoin, ethotoin, phenobarbital, phenobarbital sodium, mephobarbital, metharbital, trimethadione, ethosuximide, acetylpheneturide, primidone, sodium valproate, carbamazepine, zonisamide, acetazolamide, diazepam and the like.

An example of an anti-arrhythmic agent includes aprindine hydrochloride, amiodarone hydrochloride, 1-isoprenaline, quinidine hydrochloride, disopyramide, disopyramide phosphate, cibenzoline succinate, pirmenol hydrochloride, flecainide acetate, pilsicainide hydrochloride, procainamide hydrochloride, propafenone hydrochloride, mexiletine hydrochloride, lidocaine and the like.

An example of an antidepressant includes desipramine hydrochloride, nortriptyline hydrochloride, amoxapine, maprotiline hydrochloride, imipramine hydrochloride, amitriptyline hydrochloride, clomipramine hydrochloride, trimipramine maleate, lofepramine hydrochloride, dosulepin hydrochloride, trazodone hydrochloride, fluvoxamine maleate, paroxetine hydrochloride hydrate, milnacipran hydrochloride, mianserin and the like.

An example of an antianxiety agent includes alprazolam, etizolam, oxazolam, cloxazolam, clotiazepam, chlordiazepoxide, diazepam, fludiazepam, bromazepam, medazepam, ethyl loflazepate, lorazepam, hydroxyzine hydrochloride, hydroxyzine pamoate, flutazolam tofisopam and the like.

An example of an antipsychotic agent includes chlorpromazine hydrochloride, thioridazine hydrochloride, propericyazine, perphenazine, fluphenazine decanoate, levomepromazine maleate, spiperone, timiperone, heloperidol, haloperidol decanoate, bromperidol, clofectone, sulpiride, zotepine, pimozide, mosapramine hydrochloride, risperidone, perospirone hydrochloride hydrate, quetiapine fumarate, olanzapine and the like.

An example of an adrenocortical hormone includes dexamethasone, dexamethasone palmitate, triamcinolone acetonide, hydrocortisone, fludrocortisone acetate, prednisolone, betamethasone, methylprednisolone and the like.

An example of an antihistaminine includes clemastine fumarate, chlorpheniramine d-maleate, cyproheptadine hydrochloride, promethazine hydrochloride, homochlorcyclizine hydrochloride, mequitazine, diphenhydramine hydrochloride, ebastine, cetirizine hydrochloride, olopatadine hydrochloride, fexofenadine hydrochloride and the like.

An example of a local anesthetic includes cocaine hydrochloride, bupivacaine hydrochloride, procaine hydrochloride, mepivacaine hydrochloride, dibucaine hydrochloride, tetracaine hydrochloride, lidocaine hydrochloride, sotalol hydrochloride and the like.

An example of an NMDA antagonist includes ketamine, dextromethorphan and the like.

An example of a remedy for migraine includes dihydroergotamine mesylate, lomerizine hydrochloride, sumatriptan succinate and the like.

An example of a remedy for diabetic neuropathy with pain includes mexiletine hydrochloride and the like.

An example of a calcium channel inhibitor includes gabapentin, technotide, tregilobalin, pregabalin and the like.

Other agent(s) which supplements and/or potentiates the preventing and/or treating effect of the compound of the present invention also include(s) not only the agents which have been found up to now based on the mechanism described above but also the agents which will be found in future.

### Effect of the Invention

In the present invention, by mixing the compound (I) or a salt thereof with a non-crystallizing polymer (such as HPMCAS), it is possible to preferably provide an amorphous state having an excellent stability upon preservation (chemical and physical stability). Furthermore, by appropriately controlling the particle size of the pharmaceutical composition provided by the present invention, it is possible to make the individual difference of transition of plasma concentration small as a transnasal preparation or an oral transmucosal preparation (oral mucosal adhesive tablets or oral mucosal adhesive film preparations). Thus, absorbing property in *vivo* is able to be improved and BA is able to be improved.

Additionally, the present invention provides a transnasal preparation having improved administration compliance in which irritation in nasal cavity is reduced by controlling particle size of the composition. Furthermore, the present invention also provides an oral mucosal adhesive preparation in which irritation in nasal cavity is avoided.

### Brief Description of the Drawings

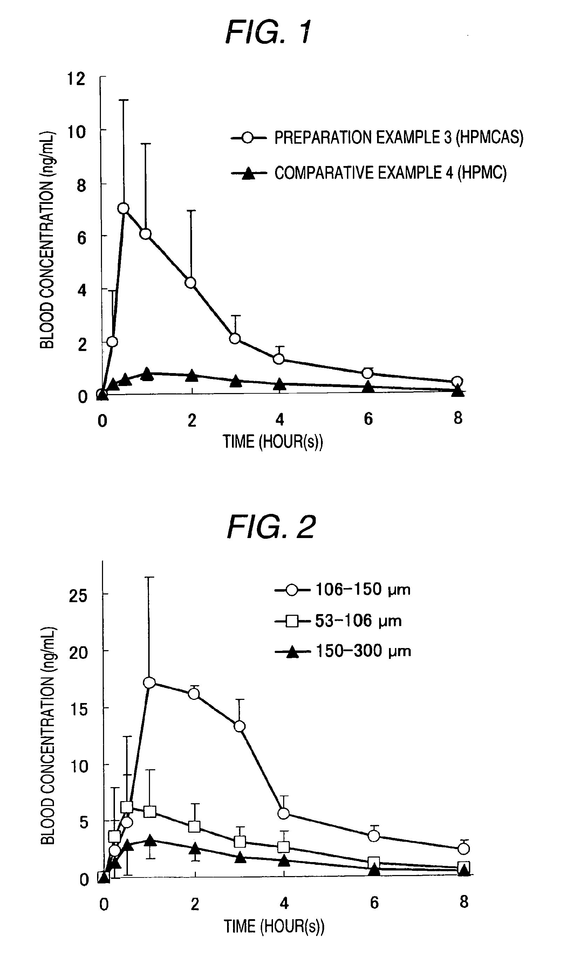

Fig. 1 shows the temporal changes in blood concentration when the preparations prepared in Comparative Example 4 and Preparation Example 3 were nasally administered. "▲" represents the preparation of Comparative Example 4 where HPMC is used while "o" represents the preparation of Preparation Example 3 where HPMCAS is used.
Fig. 2 shows the temporal changes in blood concentration of the compound (I) in each particle size distribution of the preparations. "o" represents a preparation where the particle size distribution is 106 to 150 µm, "□" represents a preparation where the particle size distribution is 53 to 106 µm and "▲" represents a preparation where the particle size is 150 to 300 µm.

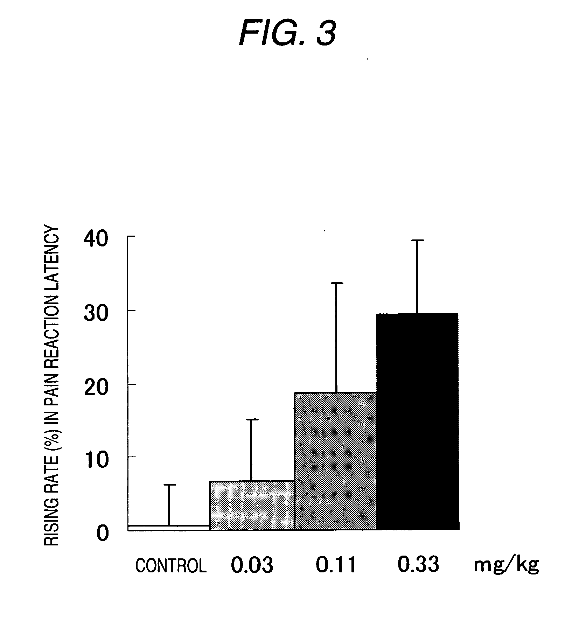

Fig. 3 shows changes in pain reaction latency when an amorphous nasal preparation (0.03 kg/kg, 0.11 mg/kg and 0.33 mg/kg) using the compound (I) and HPMCAS prepared in Preparation Example 5 and a control preparation (HPMCAS) were administered to rhesus monkeys in which spinal nerve was ligated.

### Best Mode for Carrying Out the Invention

Hereinafter, although the effect of the present invention will be described in detail by way of Preparation Examples, Comparative Examples and Test Examples, the present invention is not limited thereto but may be modified within a scope of the present invention.

### Preparation Example 1: Production of HPMCAS-containing preparation

HPMCAS (trade name: AQOAT-LF (manufactured by Shin-Etsu Chemical Co., Ltd.)) (26.7 g) was added to and dissolved in dichloromethane/anhydrous ethanol = 1/1 (v/v, 600 mL). Then the compound (I) (13.3 g) was added thereto and the resulting solution was spray-dried to give amorphous powder.

### Comparative Example 1: Production of HPMCAS-containing preparation

The compound (I) (5 g) was added to and dissolved in a mixed liquid of dichloromethane/anhydrous ethanol = 1/1 (v/v, 800 mL). Then, hydroxypropyl methylcellulose (hereinafter, referred to as HPMC) (trade name: Tc-5EW (manufactured by Shin-Etsu Chemical Co., Ltd.)) (15 g) was added thereto and the resulting solution was spray-dried to give amorphous powder.

### Comparative Example 2: Production of PVP-containing preparation

The compound (I) (15 g) was added to and dissolved in anhydrous ethanol (500 mL). Then, polyvinylpyrrolidone (hereinafter, referred to as PVP) (trade name; Kollidon 30 (manufactured by BASF Takeda Vitamin) (45 g) was added thereto and the resulting solution was spray-dried to give amorphous powder.

### Comparative Example 3: Production of an aminoacryl methacrylate copolymer-containing preparation

The compound (I) (16.67 g) was added to and dissolved in anhydrous ethanol (500 mL). Then, an aminoacryl methacrylate copolymer (hereinafter, referred to as Eudragit) (trade name: Eudragit RLPO (manufactured by Degussa) (50 g) was added thereto and the resulting solution was spray-dried to give amorphous powder.

### Preparation Example 2: Production of HPC-containing preparation

The compound (I) (5 g) was added to and dissolved in anhydrous ethanol (300 mL). Then, hydroxypropyl cellulose (hereinafter, referred to as HPC) (trade name: HPC-L (manufactured by Nippon Soda Co., Ltd.)) (15 g) was added thereto and the resulting solution was spray-dried to give amorphous powder.

The spray-drying in Preparation Examples 1 and 2 and Comparative Examples 1 to 3 was carried out under the following conditions.

Device used: Spray-Drier GS310 (manufactured by Yamato Scientific Co., Ltd.); temperature of supplying air: 120°C; temperature of exhaust air: 74 to 76°C; orifice pressure: 0.7 kPa; filter pressure: 0.3 kPa; spray pressure: 0.05 mPa; liquid flow speed: 8.3 mL/minute.

### Test Example 1: Method for conducting a stability test

Each of the amorphous powder produced in Preparation Examples 1 and 2 and Comparative Examples 1 to 3 was placed in an aluminum pillow together with silica gel and tightly sealed, followed by storing at 60°C for one month to conduct a stability test. After completion of the test, chemical stability was evaluated (purity test) and physical stability was evaluated (X-ray diffraction analysis and differential scanning calorimetry (DSC) analysis). Result of the chemical stability test is shown in the following Table 1 while that of the physical stability test is shown in Table 2.

**Table 1**

| | Initial Values | 60°C for 1 Month |
|---|---|---|
| Preparation Example 1 (HPMCAS) | 99.8 (100) | 95.1 (95.3) |
| Preparation Example 2 (HPC) | 98.7 (100) | 95.7 (97.0) |
| Comparative Example 1 (HPMC) | 96.8 (100) | 91.0 (94.0) |
| Comparative Example 2 (PVP) | 99.6 (100) | 91.2 (91.6) |
| Comparative Example 3 (Eudragit) | 100.7 (100) | 94.2 (93.5) |

**Table 2**

| Polymer | Period | Non-Crystallizing (%) | X-Ray Form |
|---|---|---|---|
| Preparation Example 1 (HPMCAS) | Initial Value | 100 | Halo |
| | 60°C for 1 Month | 100 | Halo |
| Preparation Example 2 (HPC) | Initial Value | 22 | Halo |
| | 60°C for 1 Month | 34 | Crystalline Peak |
| Comparative Example 1 (HPMC) | Initial Value | 63 | Halo |
| | 60°C for 1 Month | 60 | Partially Crystalline Peak |
| Comparative Example 2 (PVP) | Initial Value | 100 | Halo |
| | 60°C for 1 Month | 96 | Halo |
| Comparative Example 3 (Eudragit) | Initial Value | 100 | Halo |
| | 60°C for 1 Month | 0 | Crystalline Peak |

The data in Table 1 are the ratio (% by weight) of the measured values to the theoretical values and the data in the parentheses are residual rates to the initial values. Table 1 represents that the formulation prepared by a solvent evaporation method by means of spray drying suppresses a decrease in the agent content compared with the initial value by using HPC or HPMCAS as a polymer. Thus, in view of chemical stability, the water-soluble polymers as such are suitable.

"Non-crystallizing (%)" in Table 2 is a value calculated by a formula of "100 - crystallization degree (%)", which was obtained by calculating a crystallizing degree (%) from endothermic peak of melting point obtained by measurement by DSC. It means the degree of changing into amorphous form. "Halo" means a non-crystalline shape where no crystalline peak is recognized. As it is apparent to be persons skilled in the art, rate of non-crystallization is also able to be calculated from the height of crystalline peak in X-ray diffraction.

No endothermic peak of the formulation in which HPMCAS or PVP is used as a polymer was recognized in DSC. Additionally, since the halo peak was also recognized in the X-ray diffraction analysis, the formulation was able to maintain the amorphous state for one month at 60°C. Thus, in order to maintain the amorphous state, HPMCAS and PVP are suitable in view of physical stability.

Taking these results into consideration, HPMCAS and HPC which are non-crystallizing polymers are suitable as polymers which are able to ensure both chemical and physical stabilities, and HPMCAS is most suitable among them.

### Comparative Example 4: Production of HPMC-containing preparation

The compound (I) (86.7 g) and HPMC (trade name: Tc-5EW (manufactured by Shin-Etsu Chemical Co., Ltd.); 260.0 g) were mixed and kneaded with melting to make amorphous. After being roughly crushed, the product was subjected to a pinmill grind to give an amorphous preparation.

### Preparation Example 3: Production of HPMCAS-containing preparation

HPMCAS (trade name: AQOAT-LF; 30 g) was dissolved in a mixed liquid (600 mL) of anhydrous ethanol/dichloromethane = 1/1 (v/v). After sieving the solution was sieved through a sieve where openings were 300 µm, the compound (I) (10 g) was dissolved in the sieved solution, followed by spray-drying the resulting spray solution to give powder. Magnesium stearate (80 mg) was added to the spray-dried powder (8 g) and mixed using a mortar with a pestle, followed by compressing by a roller compactor to give flakes. The flakes were milled using a mortar with a pestle and a fraction of 45 to 150 µm was obtained by sieving to give a preparation.

### Test Example 2: Measurement of BA upon nasal administration of an HPMC- or HPMCAS-containing preparation

The amorphous preparation (1.392 g) of HPMC produced in Comparative Example 4 was mixed with lactose for direct tableting (8.594 g) of 75 to 106 µm fraction which was previously produced by sieving and charged in two capsules (one capsule for each nasal cavity) so as to make the dose of the compound (I) to be 0.4 mg/kg to provide an HPMC-containing preparation.

The amorphous preparation (1.406 g) of HPMCAS produced in Preparation Example 3 was mixed with lactose for direct tableting (8.594 g) of 75 to 106 µm fraction which was previously produced by sieving and charged in two capsules (one capsule for each nasal cavity) so as to make the dose of the compound (I) to be 0.4 mg/kg to provide an HPMCAS-containing preparation.

The above two preparations were administered to nasal cavities of three rhesus monkeys (3 to 5 years age) in which one capsule was administered to each nasal cavity so as to administer two capsules to both nasal cavities (dose: 0.4 mg/kg) using a device for nasal administration of nasal preparations (Jetlizer (registered trademark, manufactured by Hitachi, Ltd.)). After administration of the preparation, blood was collected from femoral vein after predetermined time to measure the concentration of the compound (I) in plasma and also to calculate the BA of each preparation.

BA represents ((AUC by nasal administration/dose of nasal administration)/(AUC by intravenous administration/dose of intravenous administration) x 100) (%) in which AUC is an area under a curve of blood concentration versus time (ng.hr/mL). The result is shown in Fig. 1. BA values of the preparations of Comparative Example 4 and Preparation Example 3 were 1.8% and 10.1%, respectively.

It is apparent that when being nasally administered, an amorphous preparation of the compound (I) using HPMCAS has a higher absorption and an excellent BA than that in the case of using HPMC.

### Test Example 3: Stability tests for HPMCAS preparation and HPMC preparation

Each of the preparations produced in Comparative Example 4 and Preparation Example 3 was weighed and charged into a glass test tube, which was covered and placed in an aluminum pillow together with silica gel and heat-sealed. They were stored in a constant-temperature vessel for stability test (60°C) for one month. After that, quantitative determination, purity test, DSC and powder X-ray diffraction measurement were carried out. The result of physical stability is shown in Table 3 while the result of chemical stability is shown in Table 4.

**Table 3**

| | Polymer in Formulation | Non-crystallizing (%) | X-ray Form |
|---|---|---|---|
| Comparative Example | HPMC | 60 | Crystalline peak |
| Preparation Example 3 | HPMCAS | 100 | Halo |

**Table 4**

| | Polymer in Formulation | Agent Amount (%) | Numbers of impurities |
|---|---|---|---|
| | | (to Initial Value) | (≥0.5%) |
| Comparative Example | HPMC | 94 | 3 |
| Preparation Example 3 | HPMCAS | 98.3 | 1 |

From the non-crystallizing rate calculated from DSC and the powder X-ray diffraction measurement, partial crystallization was recognized in the preparation (HPMC preparation) produced in Comparative Example 4 after elapse of one month at 60°C. On the other hand, no crystallization was recognized in the preparation (HPMCAS preparation) produced in Preparation Example 3 and it is apparent that the preparation using HPMCAS was better in terms of physical stability.

With regard to chemical stability, it is also apparent that the preparation using HPMCAS was better in the stability in view of the agent content and numbers of impurity shown in Table 4.

### Preparation Example 4: Production of HPMCAS amorphous preparations with various particle size distributions

HPMCAS (40 g) was dissolved in a mixture (1,000 mL) of anhydrous ethanol:acetone = 1:1 (v/v). Then the compound (I) (20 g) was further dissolved therein and the resulting solution was spray-dried and dried under reduced pressure for one night at 40°C. Avicel PH 101 (2.4 g) and magnesium stearate (100 mg) were added to the obtained spray-dried powder (7.5 g). The mixture was mixed using a mortar with a pestle and tabletted with compression to give a molded product. It was crushed using a mortar with a pestle and sieved to prepare fractions of 53 to 106, 106 to 150 and 150 to 180 µm. Magnesium stearate (10.1 mg) was added to each of the fractionated powder (1 g) to give a preparation.

### Test Example 4: Measurement of BA values by nasal administration of amorphous preparations having various particle size distribution

Each of the preparations in three kinds of particle size distributions of 53 to 106 µm, 106 to 150 µm and 150 to 180 µm (0.710 g) produced in Preparation Example 4 and lactose for direct tabletting of fractions of 53 to 106 µm, 106 to 150 µm and 150 to 180 µm (4.290 g) prepared by a previous sieving were mixed and charged in a capsule for one nose so that the dose of the compound (I) was made to be 0.4 mg/kg to give a preparation comprising two capsules.

The above preparations having three kinds of particle size distributions were administered to nasal cavities of three rhesus monkeys (3 to 5 years age) in which one capsule was administered to each nasal cavity so as to administer two capsules to both nasal cavities (dose: 0.4 mg/kg) using a device for nasal administration of nasal preparations (Jetlizer (registered trademark, manufactured by Hitachi, Ltd.)). After administration of the preparation, blood was collected from femoral vein after predetermined time to measure the concentration of the compound (I) in plasma and also to calculate the BA of each preparation. The result is shown in Table 5 and Fig. 2.

**Table 5**

| Particle Size Distribution (µm) | BA (%) |
|---|---|
| 53 to 106 | 10.8 |
| 106 to 150 | 33.1 |
| 150 to 300 | 5.9 |

The above result disclose that an amorphous composition having a particle size distribution of 106 to 150 µm has a particularly excellent BA when being nasally administered. As a result thereof, it is expected that an amorphous composition having an average particle size within a range of about 100 to about 150 µm or an amorphous composition mostly having an average particle size of such a range or, in other words, an amorphous composition having an average particle size within a range of about 70 to 150 µm also has an excellent BA value.

### Preparation Example 5: HPMCAS-containing amorphous preparation

HPMCAS (trade name: AQOAT-LF; 1 kg) and the compound (I) (0.5 kg) were dissolved in a mixed solvent (18.5 kg) of anhydrous ethanol:acetone = 1:1 (w/w) and the resulting solution was spray-dried to give powder. The spray-dried powder was mixed with a predetermined ratio of lactose, a phosphate buffer of pH 7.4 (67 mM) was added thereto and the mixture was granulated. The granulated product was dried and mixed with magnesium stearate. The granulated product was milled and sieved to give granules of a fraction of 75 to 180 µm. The granules (30.5 mg) were filled in a capsule to give an amorphous nasal preparation. With regard to a control, a formulation in which HPMCAS was added instead of the spray-dried power was used among a formulation for 0.33 mg/kg. Added amounts for the production of each preparation are shown in the following Table 6.

**Table 6**

| | Preparation for Control | Preparation of 0.03 mg/kg | Preparation of 0.11 mg/kg | Preparation of 0.33 mg/kg |
|---|---|---|---|---|
| Spray-Dried Powder | - | 0.225 | 0.75 | 2.25 |
| HPMCAS | 2.25 | - | - | - |
| Lactose | 27.86 | 29.88 | 29.36 | 27.86 |
| Phosphate Buffer | 4.5 | 4.5 | 4.5 | 4.5 |
| Magnesium Stearate | 0.3 | 0.3 | 0.3 | 0.3 |

| | | | | |
|---|---|---|---|---|
| (unit: gram(s)) | | | | |

### Test Example 5: Pharmacological test for efficacy (Suppressive activity for hyperalgesia in rhesus monkeys where spinal nerve was ligated)

Six rhesus monkeys were subjected to the test and anesthetized with appropriate amounts of ketamine and pentobarbital sodium and L7 spinal nerve was ligated using silk. The preparation produced in Preparation Example 5 (0.03 mg/kg, 0.11 mg/kg and 0.33 mg/kg (converted to the concentration of the compound (I)) and a control preparation were nasally administered to the rhesus monkeys. Then heat stimulation was applied to the back of paw of the monkeys after 60 minutes. By using the escape movement caused by that as an index for pain reaction, the reaction latent period (in second(s)) was measured. Rate of the risen reaction latent period after administration to the reaction latent period before administration was calculated as a rising rate and the result is shown in Fig. 3.

The result discloses that an amorphous composition of the compound (I) using HPMCAS suppresses the hyperalgesia by nasal administration.

### Preparation Example 6: Production of oral mucosal adhesive tablets

The amorphous composition (spray-dried powder) produced in Preparation Example 4 was tabletted to produce tablets having the compositions of Table 7.

**Table 7**

| | Tablet 1 | Tablet 2 | Tablet 3 |
|---|---|---|---|
| Compound (I) | 10 | 10 | 10 |
| HPMCAS | 20 | 20 | 20 |
| Carboxyvinyl Polymer | 8 | 8 | 8 |
| HPC | 41.2 | - | - |
| PVP | - | 41.2 | - |
| HPMC | - | - | 41.2 |
| Magnesium Stearate | 0.8 | 0.8 | 0.8 |
| Total Amount | 80 | 80 | 80 |

| | | | |
|---|---|---|---|
| (unit: mg) | | | |

### Preparation Example 7: Production of oral mucosal adhesive film preparations

The amorphous composition (spray-dried powder) produced in Preparation Example 4 and other components were uniformly dispersed in hexane to make the solid content 10% by weight, the dispersion liquid was added to the inner side of a support (PE film #9720; manufactured by 3M Health Care) and spread using a Baker type applicator (manufactured by Tester Sangyo Co., Ltd.) to make the thickness uniform. This was dried in *vacuo* at room temperature for 18 hours and the applied surface was covered with an inner side of a liner (PET film for exfoliation treatment for one side #1022; manufactured by 3M Health Care) to give a film preparation having a composition of Table 8.

**Table 8**

| | |
|---|---|
| Compound (I) | 15 |
| HPMCAS | 30 |
| HPC | 40 |
| Carboxyvinyl Polymer | 5 |
| Propylene Glycol | 10 |
| Total Amount | 100 |

| | |
|---|---|
| (unit: mg) | |

### Industrial Applicability

According to the present invention, by granulating the compound (I) with an appropriate amorphous polymer such as HPMCAS, an amorphous composition which is physically and chemically stable is able to be prepared. As a result, when the preparation containing such a composition is subjected to a nasal administration or an administration by adhering to oral mucosa, BA is improved. Thus, it is possible to provide an excellent pharmaceutical agent.

## Claims

1. A pharmaceutical composition comprising amorphous (2R)-N-(1-benzylpiperidin-4-yl)-3-cyclohexylmethylthio-2-[(4R)-3-tert-butoxycarbonylthiazolidin-4-ylcarbonylamino]propanamide or a salt thereof and a non-crystallizing polymer.

2. The composition according to claim 1, wherein the non-crystallizing polymer is hydroxypropyl methylcellulose acetate succinate or hydroxypropyl cellulose.

3. The composition according to claim 1, wherein the non-crystallizing polymer is hydroxypropyl methylcellulose acetate succinate.

4. The composition according to claim 3, wherein it is a transnasal preparation.

5. The composition according to claim 4, wherein it is powdery.

6. The composition according to claim 5, wherein an average particle size of the powder is 75 µm to 180 µm.

7. The composition according to claim 5, wherein an average particle size of the powder is 100 µm to 150 µm.

8. The composition according to claim 3, wherein it is an oral transmucosal preparation.

9. The composition according to claim 8, wherein it is an oral mucosal adhesive tablet preparation or an oral mucosal adhesive film preparation.

10. The composition according to claim 3, wherein hydroxypropyl methylcellulose acetate succinate is 100 to 350 parts by weight to 100 parts by weight of (2R)-N-(1-benzylpiperidin-4-yl)-3-cyclohexylmethylthio-2-[(4R)-3-tert-butoxycarbonylthiazolidin-4-ylcarbonylamino]propaneamide.

11. The composition according to claim 3, wherein the rate of the amorphous substance after storing at 60°C for one month is 30% to 100%.

12. The composition according to claim 3, wherein the residual rate of (2R)-N-(1-benzylpiperidin-4-yl)-3-cyclohexylmethylthio-2-[(4R)-3-tert-butoxycarbonylthiazolidin-4-ylcarbonylamino]propanamide after storing at 60°C for one month is 95% to 100%.

13. The composition according to claim 3, wherein the bioavailability of (2R)-N-(1-benzylpiperidin-4-yl)-3-cyclohexylmethylthio-2-[(4R)-3-tert-butoxycarbonylthiazolidin-4-ylcarbonylamino]propanamide is 5% to 50%.

14. The composition according to claim 3, wherein the rate of the amorphous substance after storing at 60°C for one month is 30% to 100%, the residual rate of (2R)-N-(1-benzylpiperidin-4-yl)-3-cyclohexylmethylthio-2-[(4R)-3-tert-butoxycarbonylthiazolidin-4-ylcarbonylamino]propanamide after storing at the same temperature for one month is 95% to 100% and its bioavailability is 5% to 50%.

15. The composition according to claim 3, which comprises using a spray-drying granular making method, an extruder method or mixing crush method.

16. The composition according to claim 3, wherein it is an agent for treating and/or preventing pain.

17. The composition according to claim 16, wherein the pain is neuropathic pain, cancerous pain, intractable pain, postoperative pain, acute pain, chronic pain, neuralgia or infectious pain.

18. A method for the stabilizing amorphous (2R)-N-(1-benzylpiperidin-4-yl)-3-cyclohexylmethylthio-2-[(4R)-3-tert-butoxycarbonylthiazolidin-4-ylcarbonylamino]propanamide using hydroxypropyl methylcellulose acetate succinate.

19. A method for producing a pharmaceutical composition comprising stable amorphous (2R)-N-(1-benzylpiperidin-4-yl)-3-cyclohexylmethylthio-2-[(4R)-3-tert-butoxycarbonylthiazolidin-4-ylcarbonylamino]propanamide, which comprises using hydroxypropyl methylcellulose acetate succinate.
